# EUROPÄISCHE PATENTANMELDUNG

(11) **EP 3 813 072 A1**
(43) Veröffentlichungstag der Anmeldung: **28.04.2021**
(21) Anmeldenummer: 20200887.6
(22) Anmeldetag: 08.10.2020
(51) Int. Cl.: G16H 10/60

(54) **MEDIZINGERÄT UND VERFAHREN ZUM EMPFANG VON DATEN IN EINEM MEDIZINGERÄT**

(30) Priorität: 21.10.2019 DE 102019128378
(71) Anmelder: OLYMPUS WINTER & IBE GMBH, 22045 Hamburg (DE)
(72) Erfinder: Lorenz, Karsten, 14513 Teltow (DE)
(74) Vertreter: Noack, Andreas

(57) **Zusammenfassung**

Es wird ein Medizingerät vorgestellt, mit: einer Steuerung (17), die eingerichtet ist, Funktionen des Medizingeräts zu steuern, wobei die Steuerung (17) folgendes umfasst: einen Prozessor (20), und eine Netzwerkschnittstelle (25) zum Austausch von Daten mit anderen Geräten über ein Netzwerk (6), wobei die Netzwerkschnittstelle (25) eingerichtet ist, Daten unter Anwendung eines ersten Kommunikationsprotokolls auszutauschen, und über das Netzwerk (6) empfangene Daten in ein zweites Kommunikationsprotokoll zu übersetzen, und die übersetzten Daten an den Prozessor (20) weiterzuleiten.

Das Medizingerät zeichnet sich dadurch aus, dass der Prozessor (20) eingerichtet ist, von der Netzwerkschnittstelle (25) empfangene Daten mit zulässigen und/oder erwarteten Daten zu vergleichen, und von der Netzwerkschnittstelle (25) empfangene Daten abzuweisen oder zu ignorieren, wenn diese nicht mit zulässigen und/oder erwarteten Daten übereinstimmen.

Weiterhin wird ein Verfahren zum Empfang von Daten in einem Medizingerät vorgestellt.

## Beschreibung

Die Erfindung betrifft ein Medizingerät mit einer Steuerung, die eingerichtet ist, Funktionen des Medizingeräts zu steuern, wobei die Steuerung folgendes umfasst: einen Prozessor, und eine Netzwerkschnittstelle zum Austausch von Daten mit anderen Geräten über ein Netzwerk, wobei die Netzwerkschnittstelle eingerichtet ist, Daten unter Anwendung eines ersten Kommunikationsprotokolls auszutauschen, und über das Netzwerk empfangene Daten an den Prozessor weiterzuleiten.

Weiterhin betrifft die Erfindung ein Verfahren zum Empfang von Daten in einem Medizingerät.

Moderne Medizingeräte weisen heutzutage vermehrt elektronisch gesteuerte Funktionen auf. Diese Funktionen umfassen zum einen die medizinische Gerätefunktion selbst, wie z.B. die Erzeugung von elekrochirurgischen Therapiesignalen, und zum anderen die Erfassung und Kommunikation von Betriebs- und/oder Diagnosedaten.

Zur Steuerung der entsprechenden Funktionen sind solche Medizingeräte mit einer elektronischen Steuerung ausgestattet. Der Kern einer solchen Steuerung ist zumeist ein Mikroprozessor.

Um Betriebs- und Diagnosedaten mit anderen Geräten austauschen zu können, umfasst die Steuerung zumeist auch eine Netzwerkschnittstelle. Über die Netzwerkschnittstelle können moderne Medizingeräte auch für die eigentliche medizinische Funktion relevante Steuerungsdaten empfangen, wie z.B. Parameter für neue oder angepasste Untersuchungs- oder Behandlungsverfahren.

Der Datenaustausch von Medizingeräten über ein Netzwerk erfolgt üblicherweise über standardisierte Kommunikationsprotokolle. Dadurch ist gewährleistet, dass sich Medizingeräte unterschiedlicher Art und von unterschiedlichen Herstellern ohne Probleme untereinander vernetzen lassen. Gängige Protokolle sind unter anderem Ethernet, WLAN und Bluetooth.

Für den Fall, dass der Mikroprozessor der Steuerung ein zum Datenaustausch verwendetes Protokoll nicht direkt verarbeiten kann, kann die Netzwerkschnittstelle das erste Kommunikationsprotokoll in ein zweites Kommunikationsprotokoll übersetzen, welches der Mikroprozessor verarbeiten kann.

Kommunikationsprotokolle sind zumeist auf möglichst umfangreiche Funktionalität ausgelegt. Sie ermöglichen somit nicht nur die reine Übertragung von Daten, sondern auch eine direkte Beeinflussung angeschlossener Systeme bis hin zu einer kompletten Fernsteuerung. Medizingeräte mit einer Netzwerkschnittstelle könnten daher von einem potenziellen Angreifer beeinflusst werden, was in jedem Fall zu verhindern ist.

Zum Schutz gegen unbefugte Beeinflussung über das Netzwerk verfügen gängige Betriebssysteme, die auch in Medizingeräten Verwendung finden, über Schutzfunktionen in Form einer Firewall. Allerdings weisen diese Schutzfunktionen aufgrund ihres komplexen Aufbaus zumeist Fehler auf, die von Angreifern genutzt werden können, um die Firewall zu umgehen. Um jeweils neu entdeckte Fehler zu beheben müssen die Betriebssysteme daher regelmäßig aktualisiert werden. Zumeist werden neue Fehler aber zuerst von Angreifern gefunden und ausgenutzt, so dass Medizingeräte trotz regelmäßiger Aktualisierung immer einem gewissen Angriffsrisiko ausgesetzt sind.

Es stellt sich daher die Aufgabe, ein Medizingerät bereitzustellen, welches hinsichtlich der beschriebenen Problematik verbessert ist.

Diese Aufgabe wird gemäß eines Aspekts der Erfindung gelöst durch ein Medizingerät mit einer Steuerung, die eingerichtet ist, Funktionen des Medizingeräts zu steuern, wobei die Steuerung folgendes umfasst: einen Prozessor, und eine Netzwerkschnittstelle zum Austausch von Daten mit anderen Geräten über ein Netzwerk, wobei die Netzwerkschnittstelle eingerichtet ist, Daten unter Anwendung eines ersten Kommunikationsprotokolls auszutauschen, und über das Netzwerk empfangene Daten Daten in ein zweites Kommunikationsprotokoll zu übersetzen, und die übersetzten Daten an den Prozessor weiterzuleiten, welches dadurch weitergebildet ist, dass der Prozessor eingerichtet ist, von der Netzwerkschnittstelle empfangene Daten mit zulässigen und/oder erwarteten Daten zu vergleichen, und von der Netzwerkschnittstelle empfangene Daten abzuweisen oder zu ignorieren, wenn diese nicht mit zulässigen und/oder erwarteten Daten übereinstimmen.

Hierbei kann für die Kommunikation zwischen der Netzwerkschnittstelle und dem Prozessor insbesondere ein Kommunikationsprotokoll Verwendung finden, welches nicht zur direkten Beeinflussung angeschlossener Systeme vorgesehen und/oder geeignet ist. Durch die zusätzliche Plausibilitäts- bzw. Zulässigkeitsprüfung der empfangenen Daten wird eine zusätzliche Sicherheitsebene geschaffen, so dass ein zuverlässiger Schutz gegen eine unbefugte Beeinflussung des Medizingeräts erreicht wird. Es wird quasi eine weitere Sicherheitsschranke zwischen dem Medizingerät und dem Netzwerk errichtet. Dabei wird zusätzlich zu der Beschränkung von Beeinflussungsmöglichkeiten auf den Funktionsumfang des zweiten Kommunikationsprotokolls eine weitere Beschränkung auf genau die Arten von Kommunikationsinhalten erreicht, welche durch den Hersteller des Medizingeräts vorgesehen wurden. Somit kann ein Angreifer nicht auf ggf. vorhandene Schwachstellen des zweiten Kommunikationsprotokolls zugreifen, solange entsprechende Kommunikationsinhalte nicht explizit in den zulässigen Daten enthalten sind. In einer möglichen Ausführung eines Medizingeräts gemäß der Erfindung kann der Prozessor einen Filteralgorithmus ausführen, über den der Vergleich der empfangenen Daten mit zulässigen und/oder erwarteten Daten erfolgt. Ein solcher Filteralgorithmus kann mit einfachsten Datenverarbeitungsfunktionen des Prozessors umgesetzt werden und ist somit besonders unanfällig gegen versteckte Hardware- und Firmwarefehlern des Prozessors oder Fehlern des Betriebssystems.

In einer bevorzugten Ausführung eines Medizingeräts gemäß der Erfindung kann die Netzwerkschnittstelle einen Mikrocontroller umfassen, welcher eingerichtet ist, über das Netzwerk empfangene Daten in das zweite Kommunikationsprotokoll zu übersetzen und an den Prozessor zu senden, und/oder von dem Prozessor empfangene Daten in das erste Kommunikationsprotokoll zu übersetzen und in das Netzwerk zu senden.

Der Mikrokontroller fungiert somit als Übersetzer zwischen dem ersten Kommunikationsprotokoll und dem zweiten Kommunikationsprotokoll. Hierbei werden über das Netzwerk empfangene Kommunikationsinhalte, welche beispielsweise in dem ersten Kommunikationsprotokoll vorgesehene Fernsteuerfunktionen verwenden, in unschädliche Kommunikationsinhalte übersetzt.

Das Medizingerät kann in einer möglichen Ausführungsform der Erfindung ein Gehäuse aufweisen, und die Netzwerkschnittstelle kann in dem Gehäuse angeordnet sein. Dabei kann die Netzwerkschnittstelle so in dem Gehäuse angeordnet und ausgebildet sein, dass von außerhalb des Gehäuses eine Verbindung mit der Netzwerkschnittelle hergestellt werden kann. Eine entsprechende offene Ausführung der Netzwerkschnittstelle wird durch die erfindungsgemäße Sicherheitsarchitektur der Netzwerkschnittstelle und der Steuerung ermöglicht, ohne dass hierdurch die Sicherheit der Steuerung vor unbefugter Beeinflussung geschwächt wird.

Das zweite Kommunikationsprotokoll kann in einer vorteilhaften Ausführung eines Medizingeräts nach der Erfindung ein reines Datenaustauschprotokoll sein. Mit einem solchen Kommunikationsprotokoll kann eine ungewünschte Beeinflussung des Medizingeräts praktisch vollständig ausgeschlossen werden.

Das zweite Kommunikationsprotokoll kann ein SPI Protokoll sein. Das SPI ist für eine reine Datenübertragung besonders gut geeignet.

In einer weiteren Ausführung eines Medizingeräts nach der Erfindung kann die Steuerung weiterhin ein Speicherelement umfassen, und auf dem Speicherelement kann eine Liste von erwarteten und/oder zulässigen Daten gespeichert sein. Bei einer entsprechenden Ausgestaltung des Medizingeräts ist es möglich, die auf dem Speicherelement hinterlegten zulässigen Daten an geänderte und/oder neu entwickelte Funktionen des Medizingeräts anzupassen.

Ein Medizingerät gemäß der Erfindung kann ein elektrochirurgischer Hochfrequenzgenerator und/oder Ultraschallgenerator sein.

Gemäß eines weiteren Aspekts der Erfindung wird die Aufgabe gelöst durch ein Verfahren zum Empfang von Daten in einem Medizingerät, mit den Schritten: Empfang eines Datenpakets aus einem Netzwerk unter Nutzung eines ersten Kommunikationsprotokolls, Umwandeln des Datenpakets in ein zweites Kommunikationsprotokoll, welches sich von dem ersten Kommunikationsprotokoll unterscheidet, Weiterleiten des Datenpakets an einen Prozessor, Prüfen, ob das Datenpaket zulässigen oder erwarteten Daten entspricht, und Abweisen oder Ignorieren des Datenpakets, wenn es nicht zulässigen oder erwarteten Daten entspricht.

In einer möglichen Ausführungsform eines entsprechenden Verfahrens kann das Datenpaket über einen durch den Prozessor ausgeführten Filteralgorithmus geprüft werden.

Hinsichtlich weiterer Ausführungen des Verfahrens und damit erreichbarer Vorteile und Wirkungen wird auf das oben gesagte explizit verwiesen.

Die Erfindung wird nachfolgend anhand einiger beispielhafter Figuren näher erläutert. Dabei sollen die in den Figuren dargestellten Ausführungsbeispiele lediglich zum besseren Verständnis der Erfindung beitragen, ohne diese einzuschränken.

Es zeigen:
- Fig. 1:: ein Medizingerätesystem,
- Fig. 2:: ein Medizingerät,
- Fig. 3a,b:: Verfahren zum Empfang von Daten in einem Medizingerät.

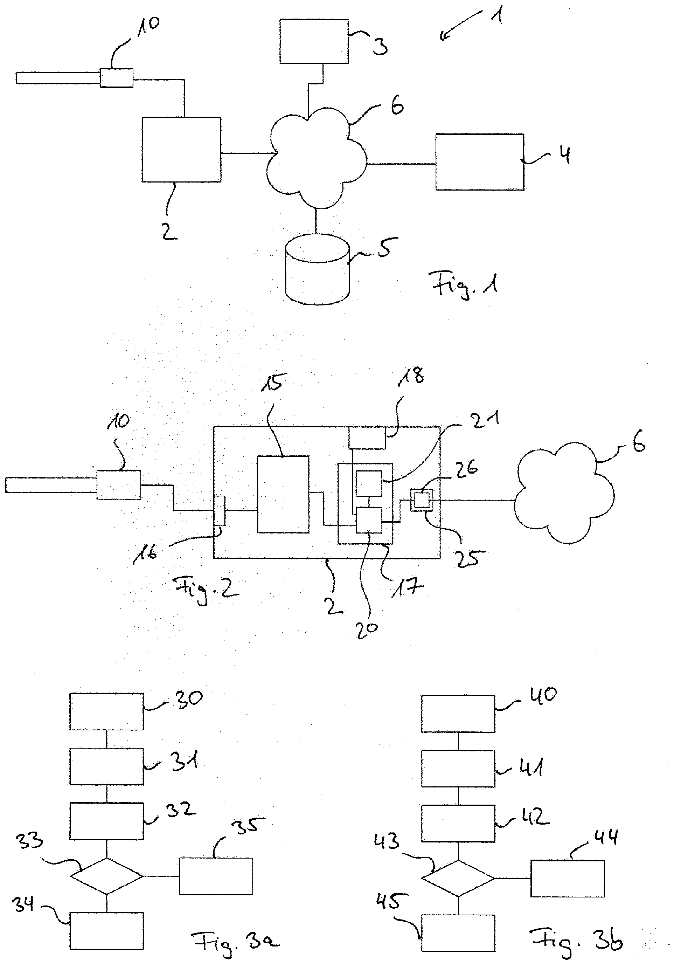

In Figur 1 ist ein Medizingerätesystem 1 mit mehreren Medizingeräten 2, 3, 4, 5 abgebildet, die über ein Netzwerk 6 verbunden sind. Das Netzwerk 6 kann ein Local Area Network (LAN) oder ein Wide Area Network (WAN) umfassen, beispielsweise das Internet.

Bei dem Medizingerät 2 handelt es sich im dargestellten Beispiel um einen elektrochirurgischen Generator, an welchen ein chirurgisches Instrument 10 angeschlossen ist.

Die weiteren Medizingeräte 3, 4, 5 können beispielsweise einen Insufflator, einen Medizingerätecontroller und/oder eine Patientendatenbank umfassen.

Die Verbindung der Medizingeräte 2, 3, 4, 5 mit dem Netzwerk 6 ist über standardisierte Kommunikationsprotokolle realisiert, beispielsweise über eine drahtgebundene Ethernet-Verbindung oder über eine drahtlose WLAN- oder Bluetooth-Verbindung.

Der prinzipielle Aufbau des Medizingeräts 2 ist in der Figur 2 schematisch dargestellt. Das Medizingerät 2 umfasst eine medizinische Funktionsbaugruppe 15, welche zum Ausführen einer medizinischen Funktion eingerichtet ist. Die Funktionsbaugruppe 15 kann beispielsweise ein chirurgischer Hochfrequenzgenerator sein.

An die Funktionsbaugruppe 15 ist das chirurgische Instrument 10 angeschlossen. Zur galvanischen Trennung eines das chirurgische Instrument 10 beinhaltenden Patientenstromkreises von einer Versorgungsspannung ist eine Trennbaugruppe 16, beispielsweise ein Trenntransformator, vorgesehen.

Das Medizingerät 2 umfasst weiterhin eine Steuerung 17, welche die Funktionsbaugruppe 15 steuert. Die Steuerung ist mit einer Benutzerschnittstelle 18 verbunden, die beispielsweise Schalter, Drehsteller, Fußpedale, und/oder einen berührungsempfindlichen Bildschirm umfassen kann. Über die Benutzerschnittstelle 18 kann ein Benutzer beispielsweise Behandlungsparameter einstellen und/oder eine Behandlung starten oder beenden.

Die Steuerung 17 umfasst einen Prozessor 20 und ein Speicherelement 21, auf welchem unter anderem Programminstruktionen für den Prozessor 20 und Steuerdaten für die Funktionsbaugruppe 15 abgelegt sind.

Der Prozessor 20 kann direkt mit dem Netzwerk 6 verbunden sein, beispielsweis mittels einer integrierten Ethernet-Schnittstelle. Hierbei tritt jedoch das Problem auf, dass das Ethernet-Protokoll umfangreiche Funktionen aufweist, um den Prozessor 20 zu beeinflussen, bis hin zu einer totalen Fernsteuerung. Die entsprechenden Funktionen greifen dabei z.T. unmittelbar in die Arbeitsweise des Prozessors 20 ein und sind daher nur schwer mittels eines Betriebssystems kontrollierbar, bei dem es sich ja lediglich um ein auf dem Prozessor 20 ablaufendes Programm handelt. Zudem ist es möglich, dass die Hardware und/oder die Firmware des Prozessors 20 Schwachstellen aufweisen, welche durch bestimmte Funktionen des Kommunikationsprotokolls ausgenutzt werden können, um die Kontrollfunktionen eines Betriebssystems zu umgehen.

Um den Prozessor 20 vor ungewollter Beeinflussung über die Ethernet-Schnittstelle zu schützen, weist das Medizingerät 2 eine separate Ethernet-Schnittstelle 25 auf, die einerseits mit dem Prozessor 20 und andererseits mit dem Netzwerk 6 verbunden ist.

Die Verbindung der Ethernet-Schnittstelle 25 und dem Prozessor 20 ist dabei über ein Kommunikationsprotokoll realisiert, welches deutlich weniger Möglichkeiten für eine ungewollte Beeinflussung des Prozessors 20 über das Netzwerk 6 bietet, als das Ethernet-Kommunikationsprotokoll. Vorzugsweise erfolgt die Kommunikation zwischen dem Prozessor 20 und der Ethernet-Schnittstelle 25 mittels eines reinen Datenprotokolls, wie z.B. dem SPI-Protokoll oder dem I²C-Protokoll.

Dazu weist die Ethernet-Schnittstelle 25 einen Mikrocontroller 26 auf, welcher über die Ethernet-Verbindung empfangene Daten in das entsprechende Datenprotokoll umwandelt, und dann an den Prozessor 20 weiterleitet. In entgegengesetzter Richtung werden Daten, die der Mikrocontroller 26 in dem Datenprotokoll von dem Prozessor 20 erhält, durch den Mikrocontroller 26 in das Ethernet-Protokoll übersetzt und an das Netzwerk 6 gesendet.

Bei der Übersetzung vom Ethernet-Protokoll in das Datenprotokoll gehen direkt auf den Prozessor 20 wirkende Steuerbefehle verloren bzw. werden in unschädliche Datenpakete übersetzt, die nicht direkt auf den Prozessor 20 wirken können.

Um eine weitere Schutzebene zwischen dem Prozessor 20 und dem Netzwerk 6 zu schaffen, läuft auf dem Prozessor 20 ein Programm, welches die aus dem Netzwerk 6 erhaltenen Datenpakete mit zulässigen bzw. erwarteten Daten vergleicht.

Entspricht ein empfangenes Datenpaket nicht den zulässigen oder erwarteten Daten, so kann das Programm die entsprechenden Daten ignorieren. Auf diese Weise werden unerlaubte Daten, mit denen ein Angreifer versuchen könnte, die Funktion des Medizingeräts 2 zu beeinflussen, ausgefiltert.

Der Vergleich von empfangenen Daten mit zulässigen Daten kann über eine sogenannte Positiv-Liste erfolgen, die in dem Speicherelement 21 hinterlegt ist. In diesem Fall sind sämtliche zulässigen Arten von Datenpaketen in der Positiv-Liste aufgelistet, und der Prozessor 20 verarbeitet nur solche Daten, die den in der Positiv-Liste abgebildeten Datenarten entsprechen. Die Datenart kann durch einen Header der Daten festgelegt sein, also eine feste Datenstruktur am Beginn eines jeden Datenpakets.

Der Vergleich von Empfangenen Daten mit zulässigen Daten kann alternativ oder zusätzlich über eine Negativ-Liste erfolgen, die in dem Speicherelement 21 hinterlegt ist. In diesem Fall sind in der Liste unzulässige Arten von Datenpaketen hinterlegt, und der Prozessor 20 verarbeitet alle Daten, die nicht einer in der Liste aufgeführten Datenart entsprechen.

In den Figuren 3a, 3b sind Verfahren zum Empfang von Daten in dem Medizingerät 2 dargestellt.

Figur 3a zeigt ein Verfahren, bei dem in einem Schritt 30 ein Datenpaket aus dem Netzwerk empfangen wird. In Schritt 31 wird das Datenpaket aus dem Ethernet-Protokoll in das SPI-Protokoll übersetzt. In Schritt 32 wird das übersetzte Datenpaket an den Prozessor 20 gesendet.

In Schritt 33 wird geprüft, ob das empfangene Datenpaket erwarteten bzw. zulässigen Daten entspricht. Dazu wird das Datenpaket mit einer Positiv-Liste verglichen, welche alle zulässigen Datenarten enthält. Entspricht das Datenpaket einer in der Positiv-Liste verzeichneten Datenart, so wird das Datenpaket in Schritt 34 durch den Prozessor 20 verarbeitet. Andernfalls wird das Datenpaten in Schritt 35 abgewiesen bzw. ignoriert.

Figur 3b zeigt ein weiteres Verfahren zum Empfang von Daten. Hier wird ein Datenpaket in einem Schritt 40 empfangen, in einem Schritt 41 aus dem Ethernet-Protokoll in das SPI-Protokoll übersetzt, und in Schritt 42 an den Prozessor 20 gesendet.

In Schritt 43 wird das Datenpaket mit eine Negativ-Liste verglichen, in denen nicht-zulässige Datenarten hinterlegt sind. Entspricht das Datenpaket einer in der Negativ-Liste hinterlegten Datenart, so wird das Datenpaket in Schritt 44 abgewiesen. Andernfalls wird es in Schritt 45 verarbeitet.

Bei der Verarbeitung von Datenpaketen in den Schritten 32 oder 44 kann der Inhalt der Datenpakete durch ein auf dem Prozessor 20 ausgeführtes Programm durchaus in Steuerbefehle umgewandelt werden, welche in die Funktionsweise des Prozessors 20 eingreifen. Dies erfolgt jedoch jederzeit unter der vollen Kontrolle des auf dem Prozessor laufenden Filteralgorithmus. Dieser kann anhand der schon beschriebenen Positiv- und/oder Negativlisten und ggf. weiterer Kriterien entscheiden, ob eine entsprechende Aktion zulässig ist. So kann der Filteralgorithmus die Ausführung einer Aktion blockieren, wenn eine übertragene Datenmenge nicht zu einer mit den Daten durchzuführenden Aktion zusammenpasst, beispielsweise wenn die übertragene Datenmenge größer ist als ein Speicherbereich, in welchen die Daten abgespeichert werden sollen. Ebenso kann der Filteralgorithmus einen Betriebszustand des Medizingeräts berücksichtigen und bestimmte Aktionen nur zulassen, wenn ein bestimmter Betriebszustand vorliegt.

## Patentansprüche

1. Medizingerät mit:
einer Steuerung (17), die eingerichtet ist, Funktionen des Medizingeräts zu steuern, wobei die Steuerung (17) folgendes umfasst:
- einen Prozessor (20), und
- eine Netzwerkschnittstelle (25) zum Austausch von Daten mit anderen Geräten über ein Netzwerk (6), wobei
- die Netzwerkschnittstelle (25) eingerichtet ist, Daten unter Anwendung eines ersten Kommunikationsprotokolls auszutauschen, und
- über das Netzwerk (6) empfangene Daten in ein zweites Kommunikationsprotokoll zu übersetzen, und die übersetzten Daten an den Prozessor (20) weiterzuleiten, **dadurch gekennzeichnet, dass**
- der Prozessor (20) eingerichtet ist, von der Netzwerkschnittstelle (25) empfangene Daten mit zulässigen und/oder erwarteten Daten zu vergleichen, und von der Netzwerkschnittstelle (25) empfangene Daten abzuweisen oder zu ignorieren, wenn diese nicht mit zulässigen und/oder erwarteten Daten übereinstimmen.

2. Medizingerät nach Anspruch 1, **dadurch gekennzeichnet, dass** der Prozessor (20) einen Filteralgorithmus ausführt, über den der Vergleich der empfangenen Daten mit zulässigen und/oder erwarteten Daten erfolgt.

3. Medizingerät nach Anspruch 1, **dadurch gekennzeichnet, dass** die Netzwerkschnittstelle (25) einen Mikrocontroller (26) umfasst, welcher eingerichtet ist,
- über das Netzwerk (6) empfangene Daten in das zweite Kommunikationsprotokoll zu übersetzen und and den Prozessor (20) zu senden, und/oder
- von dem Prozessor (20) empfangene Daten in das erste Kommunikationsprotokoll zu übersetzen und in das Netzwerk (6) zu senden.

4. Medizingerät nach Anspruch 1 oder 3, **dadurch gekennzeichnet, dass** das Medizingerät ein Gehäuse aufweist und die Netzwerkschnittstelle (25) in dem Gehäuse angeordnet ist.

5. Medizingerät nach Anspruch 4, **dadurch gekennzeichnet, dass** die Netzwerkschnittstelle (25) so in dem Gehäuse angeordnet und ausgebildet ist, dass von außerhalb des Gehäuses eine Verbindung mit der Netzwerkschnittelle (25) hergestellt werden kann.

6. Medizingerät nach einem der Ansprüche 1 bis 3, **dadurch gekennzeichnet, dass** das zweite Kommunikationsprotokoll ein reines Datenaustauschprotokoll ist.

7. Medizingerät nach einem der Ansprüche 1 bis 4, **dadurch gekennzeichnet, dass** das zweite Kommunikationsprotokoll ein SPI Protokoll ist.

8. Medizingerät nach einem der Ansprüche 3 bis 5, **dadurch gekennzeichnet, dass** die Steuerung (17) weiterhin ein Speicherelement (21) umfasst, und dass auf dem Speicherelement (21) eine Liste von erwarteten und/oder zulässigen Daten gespeichert ist.

9. Medizingerät nach einem der Ansprüche 1 bis 6, **dadurch gekennzeichnet, dass** das Medizingerät ein elektrochirurgischer Hochfrequenzgenerator und/oder Ultraschallgenerator ist.

10. Verfahren zum Empfang von Daten in einem Medizingerät, mit den Schritten:
- Empfang eines Datenpakets aus einem Netzwerk (6) unter Nutzung eines ersten Kommunikationsprotokolls,
- Umwandeln des Datenpakets in ein zweites Kommunikationsprotokoll, welches sich von dem ersten Kommunikationsprotokoll unterscheidet,
- Weiterleiten des Datenpakets an einen Prozessor (20)
- Prüfen, ob das Datenpaket zulässigen oder erwarteten Daten entspricht, und
- Abweisen oder Ignorieren des Datenpakets, wenn es nicht zulässigen oder erwarteten Daten entspricht.

11. Verfahren nach Anspruch 10, **dadurch gekennzeichnet, dass** das Datenpaket über einen durch den Prozessor (20) ausgeführten Filteralgorithmus geprüft wird.
